**Europäisches Patentamt**

(19) **European Patent Office**

**Office européen des brevets**

(11) Publication number: **0 316 964 B1**

(12) # EUROPEAN PATENT SPECIFICATION

(45) Date of publication of patent specification:
**29.05.91 Bulletin 91/22**

(51) Int. Cl.⁵: **C07D 493/04,**
**// (C07D493/04, 317:00,**
**307:00)**

(21) Application number: **88120946.4**

(22) Date of filing: **24.09.85**

(54) **Process for the purification of podophyllotoxin.**

The file contains technical information submitted after the application was filed and not included in this specification

(30) Priority: **26.09.84 GB 8424269**

(43) Date of publication of application:
**24.05.89 Bulletin 89/21**

(45) Publication of the grant of the patent:
**29.05.91 Bulletin 91/22**

(84) Designated Contracting States:
**AT BE CH DE FR GB IT LI LU NL SE**

(56) References cited:
**CHEMICAL ABSTRACTS, vol. 44, 1950, abstract no. 9633d, Columbus, Ohio, US; T.R. SESHADRI et al.: "Components of Indian podophyllum"**

(61) Publication number of the earlier application in accordance with Art. 76 EPC: **0197219**

(73) Proprietor: **pHarma-medica a-s**
**15-19 Vesterlundevej**
**DK-2730 Herlev (DK)**

(72) Inventor: **Buchardt, Ole**
**Sondersgaardsvej 73**
**DK-3500 Vaerlose (DK)**

(74) Representative: **Ritter, Stephen David et al**
**Mathys & Squire 10 Fleet Street**
**London EC4Y 1AY (GB)**

## Description

The invention relates to a new method for the purification of podophyllotoxin.

Podophyllotoxin is a highly biologically active substance which is particularly useful as a drug against condylomata acuminata (veneral warts) [G. Gabriel and R.N.T. Thin, Br.J.Vener.Dis., 59, 124-126 (1983) as well as possessing cytostatic [G. von Krogh and H.I. Maibach, Dermatologica, 167, 70-77 (1983)] and antiviral properties [T. Markkanen, M.L. Makinen, J. Miettinen, E. Maunuksela, T. Laijoki and J. Paranko, Drugs Exp.Clin.Res. 9, 1-7 (1983)].

Previously, podophyllotoxin has been isolated from the above-mentioned resins by various laborious procedures, all of which included solvent extraction, as a rule with chloroform, followed by further purification steps. According to the most authoritative newer literature [W.M. Hearon and W.S. MacGregor, Chem.Rev., 55, 1002 (1955) ; J.L. Hartwell and W.E. Detty, J.Am.Chem.Soc., 246-253 (1950)] chromatographic purification was necessary in order to obtain podophyllotoxin. In many cases, benzene has been used in the course of the purification procedures despite the acknowledged health hazards which arise. Furthermore, chromatography has previously been regarded as being absolutely essential in order to obtain podophyllotoxin in a degree of purity such that the podophyllotoxin is only slightly contaminated with the many congeners which exist in the plant material. This has resulted in pure podophyllotoxin being a rare and expensive substance which is difficult to obtain in large amounts.

There has recently been a heightened demand for pure podophyllotoxin, both as a raw material for conversion to various pharmacologically active derivatives and in clinical treatments using podophyllotoxin itself. In both cases it is highly desirable to use as pure a product as possible.

According to EP 85306787.4 (EP-A-0 197 219) there is provided a process for obtaining purified podophyllotoxin from a solution thereof which comprises contacting the podophyllotoxin solution with an aromatic or heteroaromatic compound selected from toluene, o-xylene, m-xylene, p-xylene, anisole, chlorobenzene, aniline, pyridine, phenol, nitrobenzene, quinoline, isoquinoline, furfuryl alcohol, naphthalene and benzyl alcohol so as to form a solid complex, separating the solid complex from the solution and isolating purified podophyllotoxin from the separated complex.

The process is well suited for the purification of podophyllotoxin in large quantities, it avoids the strongly carcinogenic solvent benzen, and it gives a very high yield of pure podophyllotoxin.

By repetition of this procedure with various complexing agents, 92-95% pure podophyllotoxin can be obtained.

It is often found however that several of its congeners remain as persistent impurities, even after as many as 5-8 recrystallizations using various solvents and complexing agents and to achieve an even higher degree of purity a further process step (which itself forms the subject of the present invention) may be employed.

Thus according to the invention, there is provided a process which enables podophyllotoxin of especially high purity to be obtained "absolute" purification can be performed in a very surprising and unforeseeable manner. The process comprises extraction of a solution of the impure podophyllotoxin in a water-immiscible solvent, for example chloroform, with aqueous base. This result is surprising because according to the literature, podophyllotoxin is very labile under basic conditions, being transformed into its epimer, picropodophyllin when treated with alkali [W.Borsche and J. Niemann, Liebiegs Ann.Chem. 494, 126-142 (1932)].

The following Examples illustrate the process of the invention.

## Example 1.

A. Podophyllin (643 g) is dissolved in ethanol (1.5 l) with gentle heating. To the hot solution is added toluene (643 ml) followed by water (129 ml). The solution is cooled with ice-water and the precipitated crystalline material isolated by filtration and washed with ether (2 l). Yield : 342.8 g.

This material is a complex of podophyllotoxin, toluene and water, and it was shown by HPLC to contain ca. 13% of related lignans.

The complex was redissolved in ethanol (1.0 l) with gentle heating, pyridine (140 ml) followed by water (470 ml) were added, and the solution was cooled with ice-water. The precipitated crystalline material was isolated by filtration and washed with ether (250 ml). Yield : 292 g.

This material consists of a podophyllotoxin-pyridine-water complex which was shown by HPLC to contain ca. 7% of other related lignans. The material was recrystallized from ethanol (1.0 l)-water (430 ml) to give 239 g of the pyridine-water complex.

B. The complex (119 g) was dissolved in chloroform (1.2 l) which was treated with : (i) 0.8 N HCl (500 ml), (ii) 0.4 N NaOH (500 ml), (iii) 0.01 N HCl (450 ml), (iv) water (450 ml), and (v) saturated aqueous sodium chloride (500 ml).

The chloroform solution was dried over solid magnesium sulfate, the chloroform was removed, in vacuo, and the remaining sirupy material was recrystallized from ethyl acetate (450 ml) to give podophyllotoxin, aq. (50 g). This material was shown by HPLC to contain < 0.4% of other lignans. A sample was subjected to drying (100°C), in vacuo, to give analytically pure podophyllotoxin. Anal. Found (cal.) C 63.62 (63.76), H 5.39 (5.35). Its IR, $^1$HNMR and $^{13}$C NMR spectra were identical with those recorded in the literature.

Example 2.

A. Podophyllin (1 kg) was dissolved in ethanol (3 l) with gentle heating. As soon as the solution was homogeneous, toluene (1.0 l) and water (200 ml) were added and the mixture was cooled with ice-water. The precipitated crystalline material was filtered off, and washed with ether (1.0 l). This was subsequently recrystallized from ethanol (2 l), toluene (650 ml) and water (130 ml). The isolated crystalline material was redissolved in ethanol (1.5 l), pyridine (210 ml) and water (630 ml) were added, and the solution was cooled with ice-water. The precipitated crystalline complex was isolated, washed with ether (1.0 l) and dried to give 478 g of material.
The product of 2A was dissolved in chloroform (5 l) and treated with : (i) 0.4 N HCl (2 l), (ii) 0.2 N NaOH (2 l), (iii) 0.03 N HCl (1. 4 l) (iv) saturated aqueous sodium chloride (700 ml), and (v) dried over magnesium sulfate. The chloroform was removed, in vacuo, and the remaining sirupy material recrystallized from ethanol-water (1.4 l-0.9 l). This yielded podophyllotoxin, $2H_2O$ (419 g, ~ 39%) which contained < 0.6% picropodophyllin and < 0.1% of other related lignans.
Anal. Found (calc.) C 58.63 (58.67), H 5.53 (5.77). Identified by IR and $^1$HNMR spectroscopy. A small sample was dried (100°C), in vacuo, to give an analytically pure sample. Anal. C 63.75 (63.76), H 5.41 (5.35).

Example 3

A. Podophyllin (500 g) was dissolved in ethanol (1.5 l) with gentle heating, toluene (0.5 l) and water 100 ml) were added, the solution was cooled, and the precipitated crystalline material was isolated by filtration and washed with ethanol-toluene-water (15 : 5 : 1, 200 ml). This was immediately redissolved in ethanol (1.0 l), toluene (330 ml) and water (66 ml), and the solution was cooled with ice-water. The precipitate was isolated as described above and dried to give 211 g. This was redissolved in boiling methanol (500 ml) and the hot solution was filtered, water (368 ml) was added, and the solution was cooled with ice-water to give after filtration and drying, in vacuo, over $H_2SO_4$, and at ambient pressure at 100°C, 192 g of podophyllotoxin and other related lignans in a ratio of 9 : 1.
B. The above impure podophyllotoxin (5 g) was dissolved in chloroform (50 ml) and treated successively with : (i) 0.4 N NaOH (50 ml), (ii) 0.02 N HCl (50 ml), (iii) water (50 ml), (iv) saturated sodium chloride (50 ml), the chloroform was removed, in vacuo, and the remaining material was recrystallized from ethanol-water (1 : 1, 20 ml). This gave podophyllotoxin, aq., identified by IR spectroscopy, which contained < 1.4% of other lignans.

Example 4.

A. Podophyllin (50 g) was dissolved in ethanol (150 ml) with heating, followed by addition of chlorobenzene (50 ml) and water (10 ml). After cooling, the precipitate was isolated by filtration and washed with ether (20 ml). Yield of podophyllin-chlorobenzene-water complex : 19.5 g, which was shown to contain ca. 13% of other lignans.
The above material (5 g) was redissolved in ethanol (15 ml), phenol (5 g) and water (2 ml) were added, the solution was cooled, and the precipitated material was removed by filtration and washed with ether (10 ml). Yield of podophyllotoxin-phenol-water complex : 4.2 g.
B. This was dissolved in ethyl acetate (20 ml) and the solution was treated with 2 N NaOH (20 ml). The organic phase was separated and concentrated, in vacuo. The resulting material was recrystallized from ethanol-water (1 : 1, 20 ml) to give, after drying, in vacuo, over $H_2SO_4$ : 3.75 g of > 98.5% pure podophyllotoxin, aq., determined by HPLC and identified by IR, $^1$H NMR and $^{13}$C NMR spectroscopy, $[\alpha]_D = -118.5°$.

Claims

1. A process for removing impurities from a solution comprising impure podophyllotoxin and a water-immiscible organic solvent which comprises contacting the solution with an aqueous base and isolating purified podophyllotoxin from the solution.

2. A process according to Claim 1 wherein the base is an alkali metal hydroxide.

Ansprüche

1. Verfahren zur Entfernung von Verunreinigungen aus einer Lösung, die unreines Podophyllotoxin und ein mit Wasser nicht mischbares organisches Lösungsmittel enthält, das daraus besteht, daß man die Lösung mit einer wässrigen Base in Kontakt bringt und das gereinigte Podophyllotoxin aus der Lösung isoliert.

2. Verfahren nach Anspruch 1, worin die Base ein Alkalihydroxid ist.

## Revendications

1. Procédé pour éliminer des impuretés d'une solution comprenant de la podophyllotoxine impure et un solvant non miscible à l'eau, caractérisé en ce qu'il comprend la mise en contact de la solution avec une base aqueuse et la séparation de la podophyllotoxine à partir de la solution.

2. Procédé selon la revendication 1, caractérisé en ce que la base est un hydroxyde de métal alcalin.